# EUROPÄISCHE PATENTSCHRIFT

(11) **EP 1 467 681 B1**
(45) Veröffentlichungstag und Bekanntmachung des Hinweises auf die Patenterteilung: **20.02.2008**
(21) Anmeldenummer: 02791820.0
(22) Anmeldetag: 13.12.2002
(51) Int. Cl.: A61F 2/36, A61F 2/40

(54) **HÜFT- UND SCHULTERGELENK-ENDOPROTHESE**
HIP JOINT AND SHOULDER JOINT ENDOPROTHESIS
ENDOPROTHESE D'ARTICULATION DE LA HANCHE ET DE L'EPAULE

(30) Priorität: 14.12.2001 DE 20120241 U
(43) Veröffentlichungstag der Anmeldung: 20.10.2004
(73) Patentinhaber: Mathys AG Bettlach, 2544 Bettlach (CH)
(72) Erfinder: GLIEN, Wilfried, 07639 Bad Klosterlausnitz (DE); DALLMANN, Frank, 04626 Schmölln (DE); SALOMON, Dirk, 04626 Jonaswalde (DE); KÄPPEL, Lars, 07819 Triptis (DE); OBERBACH, Thomas, 07629 Reichenbach (DE); KATZER, Thomas, 07629 Hermsdorf (DE)
(74) Vertreter: Körfer, Thomas
(86) Internationale Anmeldenummer: PCT/EP2002/014214
(87) Internationale Veröffentlichungsnummer: WO 2003/051238

(56) Entgegenhaltungen:
- EP-A- 0 009 148
- EP-A- 0 028 546
- EP-A- 0 051 729
- WO-A-01/67988
- WO-A-98/07393
- DE-A- 2 751 537
- DE-A- 3 923 154
- DE-A- 4 220 217
- DE-A- 19 834 277
- DE-A- 19 904 126
- FR-A- 995 762
- FR-A- 1 063 680
- FR-A- 2 686 503
- GB-A- 719 308
- US-A- 4 042 980
- US-A- 5 571 203

## Beschreibung

Die Erfindung betrifft eine Hüft- und Schultergelenk-Endoprothese mit einem Schaft und einer Kopfkalotte, die einteilig mit dem Schaft ist oder am Schaft befestigbar ist. Die Kopfkalotte hat eine gewölbte Oberseite und eine geschlossene Unterseite. Der Schaft und die Kopfkalotte haben jeweils eine Längsachse und die Achse der Kopfkalotte verläuft durch den Kalottenmittelpunkt und etwa senkrecht zur Kalottenunterseite.

Eine Hüft - oder Schultergelenk - Endoprothese gemäß Oberbegriff von Patentanspruch 1 ist z.B. aus DE-A-3 923 154 bekannt.

Konventionelle Endoprothesen werden über einen in einem bestimmten Winkel (z.B. 135*) zur Kopfachse geschwenkten Schaft intramedulär verankert, wobei der Schaft eine Länge von 10 bis 20 cm hat und in den Markhohlraum des Knochens eingesetzt wird. In Abhängigkeit von der Gestaltung der Prothese und davon, ob eine zementfreie oder zementierte Implantation vorgenommen wird, kann es zu einer Überbestimmung im Verankerungsmechanismus kommen. Stützt sich die Prothese mit ihrer distalen Spitze intramedulär ab, wird die natürliche Krafteinleitung von der proximalen Kortikalis im Kalottenbereich weg in den intramedulären Schaftbereich umgeleitet. Dadurch entstehen Knochenumbildungen, d.h. Knochenzuwachs um das distale Prothesenende und Knochenrückbildungen im Grenzbereich zwischen Kopfkalotte und Epiphyse (proximaler Knochen). Dies wird häufig als Ursache für Lockerungen angeführt. Es besteht auch die Gefahr eines diaphysären Schaftbruchs im Bereich des distalen Prothesenendes entweder aufgrund einer schlagartigen Überbelastung bei distaler Abstützung der Prothese oder bei proximaler Abstützung der Prothese infolge von Knochenabbau im distalen Prothesenbereich.

Die Reproduktion der anatomischen Ausgangssituation wird erschwert durch signifikante Streuungen von Inklination und Retrotorsion der Kopfkalotte zum Schaft. Wenn sich der Prothesenschaft bei der Implantation zwangsläufig in der Diaphyse ausrichtet, liegt die Kopfkalotte bei Prothesen mit fest eingestellter Inklination und Retrotorsion auch trotz aufwendiger Instrumentierung oft nicht flächig auf der Resektionsebene auf. Die daraus resultierende inhomogene Krafteinleitung führt zu Knochenumbildungen und letztlich zu Lockerungen. Neuere Prothesengenerationen insbesondere in der Schulterprothetik ermöglichen die Einstellung genannter Parameter, allerdings mit hohem technischen Aufwand.

Zur Lösung dieses Problems ist es bereits bekannt, den Schaft wesentlich kürzer auszubilden und nicht in den Markhohlraum des Knochens einzusetzen, sondern einen kurzen Schaft zu verwenden und diesen parallel zur Achse der Kopfkalotte auszurichten. Bei einer solchen aus DE 198 34 277 C2 bekannten Hüft-Schenkelhalsendoprothese ist neben der Abstützung auf der Resektionsebene zusätzlich über einen lateralen kortikalen Durchbruch eine axiale Führung und Fixierung vorgesehen. Die Verankerung erfolgt über eine Fixierung an den beiden Enden des Schaftes.

US 4,042,980 zeigt eine Humeruskomponente mit Kurzschaft, bei der die Kopfkalotte hohl oder geschlossen sein kann. Der Schaft weist umlaufende Rippen auf.

DE 37 07 518 A1 zeigt eine Prothese mit einer Vielzahl von Verankerungspfeilern, die mit unterschiedlichen Krümmungen von der Resektionsebene in Richtung Schaft verlaufen.

DE 196 13 078 A1 beschreibt eine Verankerungstechnik mittels Käfigstruktur, in deren Zwischenräume Spongiosa einwachsen kann. Diese Struktur bzw. der nach distal ausgebildete Schaft ist ebenfalls ausgehend von der Kalottenachse zur Schaftachse geschwenkt und reicht bis in den diaphysären Schaftbereich.

DE 42 20 217 A1 beschreibt eine Prothese zum Ersatz der Gelenkoberfläche, insbesondere der Knorpelschicht. Demnach ist die Kalotte nicht massiv ausgebildet und schließt nicht mit einer ebenen Fläche ab, sondern ist kappenförmig im Inneren ausgenommen. Bei derartigen Cup-Prothesen besteht die Gefahr einer Osteolyse

Bekannt ist ferner die von der Firma ESKA Implants GmbH & Co in Verkehr gebrachte Schulter-Endoprothese Typ Rozing, die modular aufgebaut ist und in der kürzesten Ausbaustufe auf eine rein proximale Verankerung ausgelegt ist. Hier ist allerdings die Schaftachse gegenüber der Kalottenachse um einen festen Inklinationswinkel geschwenkt, um in längeren Ausbaustufen eine intrameduläre Verankerung zuzulassen. Das zieht die oben erwähnte Überbestimmung bei der Verankerung wie bei konventionellen Langschaftprothesen nach sich.

Die Aufgabe der vorliegenden Erfindung ist eine Hüft- oder Schultergelenk-Endoprothese, die sich mit einfachen Mitteln exakt positionieren und dauerhaft fixieren lässt.

Erfindungsgemäß wird diese Aufgabe durch die Merkmale des Patentanspruchs 1 gelöst. Die Längsachse des Schaftes schlieft einen Winkel von maximal 30* mit der Achse der Kopfkalotte, der Konuswinkel des Schafts beträgt 5° bis 30° und die Kopfkalotte hat die Form eines Kugelabschnitts von weniger als einer Halbkugel und die Länge des Schaftes ist kleiner als der Durchmesser der Kopfkalotte.

Vorzugsweise beträgt der Konuswinkel des Schafts etwa 20°.

Die erfindungsgemäße Kurzschaftprothese wird ausschließlich in der Epiphyse verankert, wobei die Schaftachse gegenüber der Achse durch den Kalottenmittelpunkt um maximal 30°, vorzugsweise um maximal 20°, geschwenkt ist. In einer besonders bevorzugten Ausführungsform fällt die Schaftachse mit der Achse der Kopfkalotte zusammen. Dadurch wird eine Überbestimmung bei der Verankerung vermieden und wird die Krafteinleitung über den kortikalen Rand der Resektionsebene sowie mittels Pressfit in der Spongiosa sichergestellt. Zur Vermeidung eines lateralen Durchbruchs ist der Schaft sehr kurz, vorzugsweise kürzer als der Durchmesser der Kopfkalotte.

Kopfkalotte und Schaft können einteilig oder zweiteilig ausgebildet sein. Bei einer zweiteiligen Ausbildung sind der Schaft und die Kopfkalotte getrennte Bauteile, die miteinander verbindbar sind. Die Verbindung kann durch Formschluss, z.B. Schnappelemente, oder durch Formkraftschluss, z.B. eine Gewindeverbindung, erfolgen. Vorzugsweise erfolgt die Verbindung von Schaft und Kopfkalotte über einen konischen Ansatz am Schaft und eine entsprechende konische Ausnehmung in der Kopfkalotte. Die Anordnung kann auch umgekehrt sein und der konische Ansatz kann an der Kopfkalotte und die konische Ausnehmung im Schaft ausgebildet sein. An dem der Kopfkalotte zugewandten Ende des Schafts ist vorzugsweise eine sich scheibenförmig verbreiternde Stützfläche vorgesehen, die auf der Resektionsebene aufliegt.

Die Unterseite der Kopfkalotte oder der Stützfläche ist geschlossen und kann eben, leicht konvex oder leicht konkav sein. Insbesondere bei konvexer Ausbildung der Unterseite ergibt sich eine sehr homogene Verteilung der von dem Gelenk ausgehenden Belastung auf die Kortikalis und die Spongiosa.

Bei der erfindungsgemäßen Endoprothese ergibt sich eine optimale Reproduktion des Drehpunktes, wodurch eine Krafteinleitung erzielt wird, die weitgehend der ursprünglichen anatomischen Situation entspricht. Bei herkömmlichen Langschaftsystemen gibt dagegen der intramedulär verankerte Schaft einen bestimmten Bereich des Drehpunktes der Kopfkalotte vor. Damit kann oft der anatomischen Streuung des Sitzes der Kopfkalotte mit ihrem Drehpunkt nicht entsprochen werden, so dass es zu einer Krafteinleitung kommt, die von der ursprünglichen anatomischen Situation abweicht. Auch mit sehr aufwändigen, einstellbaren Prothesen läßt sich dies nicht vermeiden, oder nur mittels einer komplizierten Operationstechnik.

Ausgehend von der Unterseite der Kopfkalotte bzw. der Stützscheibe verjüngt sich der Schaft vorzugsweise konisch oder über einen Radius in Richtung der Schaftspitze. Der sich dabei unmittelbar an die Unterseite der Kopfkalotte bzw. der Stützscheibe anschließende Bereich kann dabei auch zylindrisch ausgebildet sein.

Zur Rotationssicherung weist der Schaft vorzugsweise eine oder mehrere Rippen auf, die sich im Allgemeinen radial um die Längsrichtung des Schaftes erstrecken. Zur Rotationssicherung kann der Schaft auch prismatisch mit glatten Außenflächen ausgebildet sein.

Die durch die Erfindung erzielbaren Vorteile bestehen insbesondere darin, dass die von dem Gelenk ausgehenden Belastungen homogen auf die Kortikalis und die Spongiosa übertragen werden, so dass hier kein Knochenabbau oder -umbau stattfindet. Durch die Konizität des Schaftes mit einem Winkel von bis zu 30°, vorzugsweise etwa 20°, wird ein sicherer Sitz des Schaftes in der Spongiosa durch Klemmung erreicht, was auf dem vorliegenden Gebiet als "Pressfit" bezeichnet wird.

Die Schaftachse liegt vorzugsweise auf derselben Achse, die durch den Kalottenmittelpunkt senkrecht zur Kalottenabschlussfläche oder -unterseite verläuft. Die Abstützung und Krafteinleitung erfolgt also über den kortikalen Ring in der Resektionsebene sowie über Pressfit in die Spongiosa bzw. in den Zementköcher bei Zementierung. Die Auflage des Kopfes über die Kalottenabschlussfläche bzw. der Stützfläche des Schaftes auf der Resektionsebene ist auch nach ungenauer Resektion und Schaftsitzeröffnung sowie Retrotorsions- und Inklinationsschwankungen flächig und gewährleistet damit eine homogene Krafteinleitung nahe der präoperativen Ausgangssituation. Damit wird die Gefahr einer Osteolyse vermindert, insbesondere im Vergleich zu Oberflächenersatzprothesen mit Innen ausgenommenen, kappenförmigen Kalotten.

Bei der Implantation der erfindungsgemäßen Endoprothese in der Schulter wird die Gelenkkalotte des Humerus vollständig resiziert. Dadurch wird der notwendige Freiraum zur Versorgung oder Implantation des Glenoids geschaffen.

Die Reproduktion des medio-dorsalen Offsets der Kopfkalotte zum Schaft kann mit einer genauen Instrumentierung bei der Eröffnung des Schaftkanals sichergestellt werden.

Um das Einwachsen des Knochengewebes zu ermöglichen, können in dem Schaft in beliebiger Anordnung eine oder mehrere Ausnehmungen vorgesehen sein, z.B. axial, radial oder schräg verlaufenden Fräsungen, Bohrungen oder Langlöcher. Es können mehrere Zapfen auf einem äußeren Ring um die Schaftachse zur Verankerung und Aufnahme von Schubkräften und zur Rotationssicherung beitragen. Die Unterseite der Kopfkalotte oder Stützfläche ist vorzugsweise strukturiert, z.B. mit Rillen versehen oder porös beschichtet.

Das Schaftteil kann aus Titan- oder Kobalt-Chrom-Legierungen und der Gelenkkopf kann aus einer Kobalt-Chrom-Legierung oder Keramik hergestellt sein. Schaftteil und/oder Gelenkkopf können ebenfalls aus Kunststoff, unverstärkt oder faserverstärkt, hergestellt sein.

Bei der endoprothetischen Versorgung von Gelenken werden sog. Cup-Prothesen als Oberflächenersatz für einen angegriffenen Gelenkkopf eingesetzt. Die Kopfkalotte ist dabei im Allgemeinen noch relativ gut erhalten, so dass die Endoprothese im Prinzip eine halbkugelförmige Kappe ist, die auf den Gelenkkopf aufgesetzt wird. Ist der Gelenkkopf dagegen weitgehend zerstört, so wird üblicherweise eine Langschaftprothese eingesetzt. Die erfindungsgemäße Gelenk-Endoprothese ist in vielen Fällen eine Alternative zu derartigen Langschaftprothesen, aber auch zu den erwähnten Kurzschaftprothesen mit kappenförmigen Kalotten, und stellt ein bisher in dieser Konsequenz nicht umgesetztes Konzept in der Schulterendoprothetik dar. Lediglich bei Gelenkfrakturen kann häufig auf eine Langschaftprothese nicht verzichtet werden.

Ausführungsbeispiele der Erfindung werden nachfolgend anhand der Zeichnung erläutert. Es zeigen:
- Fig. 1 bis 3: eine einteilige Gelenk-Endoprothese in einer Darstellung von unten und von der Seite bzw. in einer räumlichen Darstellung;
- Fig. 4 bis 6: ein zweites Ausführungsbeispiel einer einteiligen Gelenk-Endoprothese von unten, von der Seite bzw. in einer perspektivischen Darstellung;
- Fig. 7 bis 10: ein Ausführungsbeispiel einer zweiteiligen Gelenk-Endoprothese von unten, von der Seite in auseinandergezogenem Zustand, von der Seite in zusammengesetztem Zustand bzw. in einer perspektivischen Darstellung;
- Fig. 11 bis 14: ein zweites Ausführungsbeispiel einer zweiteiligen Gelenk-Endoprothese von unten, von der Seite in auseinandergezogenem Zustand; in der Seite im zusammengesetzten Zustand bzw. in einer perspektivischen Darstellung;
- Fig. 15 bis 18: den Schaft einer zweiteiligen Gelenk-Endoprothese von unten, von der Seite, im Längsschnitt bzw. in einer perspektivischen Darstellung;
- Fig. 19 und 20: zwei Ausführungsformen der Gelenk-Endoprothese im Schnitt;
- Fig. 21 und 22: eine Ausführungsform der Gelenk-Endoprothese mit einem unter einem Winkel angesetzten Schaft;
- Fig. 23: eine Ausführungsform mit gerillter Unterseite der Kopfkalotte und
- Fig. 24: im Schnitt die implantierte Gelenk-Endoprothese.

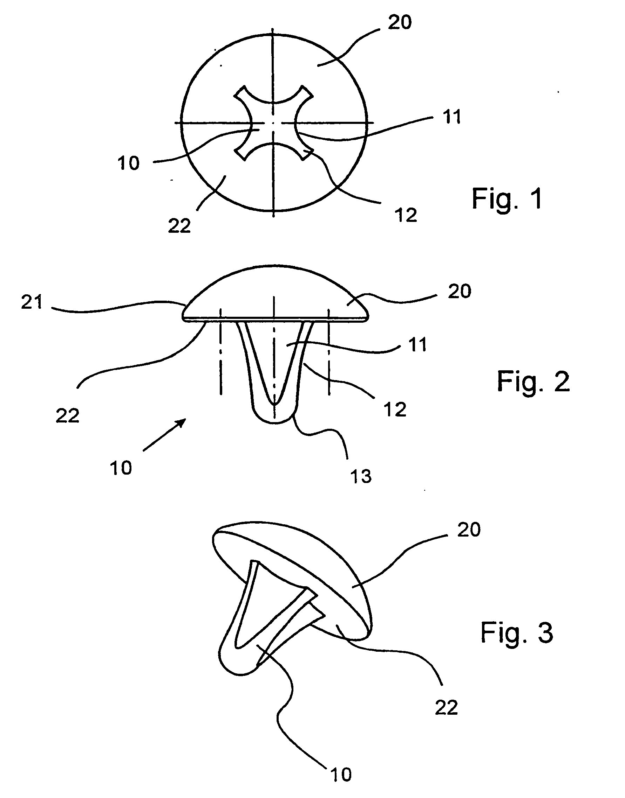

Die Fig. 1 bis 3 zeigen eine einteilige Gelenk-Endoprothese mit einem Schaft 10 und einer Kopfkalotte 20.

Der Schaft 10 ist mit vier Rillen 11 kanneliert, zwischen denen jeweils Rippen 12 stehen bleiben. Die Außenkanten der Rippen 12 sind leicht konkav gekrümmt. Nach unten verjüngen sich die Rippen 12 und enden in einer abgerundeten Spitze 13. Die Außenkante jeder Rippe'12 verläuft unter einem Winkel von etwa 10° zur Längsachse des Schafts 10. Die Fläche der Rillen 11 ist dagegen axial ausgerichtet.

Die Kopfkalotte 20 hat eine gewölbte Oberseite 21 und eine ebene Unterseite 22 und hat damit insgesamt die Form eines Kugelabschnitts, wobei der Kugelabschnitt weniger als eine Halbkugel ausmacht. Der Zentrumswinkel α beträgt weniger als 170°.

Die Querabmessung der Kopfkalotte 20 kann bspw. 43 mm betragen und die Länge des Schafts 10 dagegen nur 25 mm. Durch die Kannelierung des Schafts 10 sitzt die Endoprothese rotationssicher. Durch die sich zur stumpfen Spitze 13 hin verjüngenden Rippen 12 ergibt sich eine Art von Presssitz der Prothese in der Spongiosa (Pressfit). Die Unterseite 22 der Kopfkalotte 20 sitzt flächig auf der Resektionsebene auf.

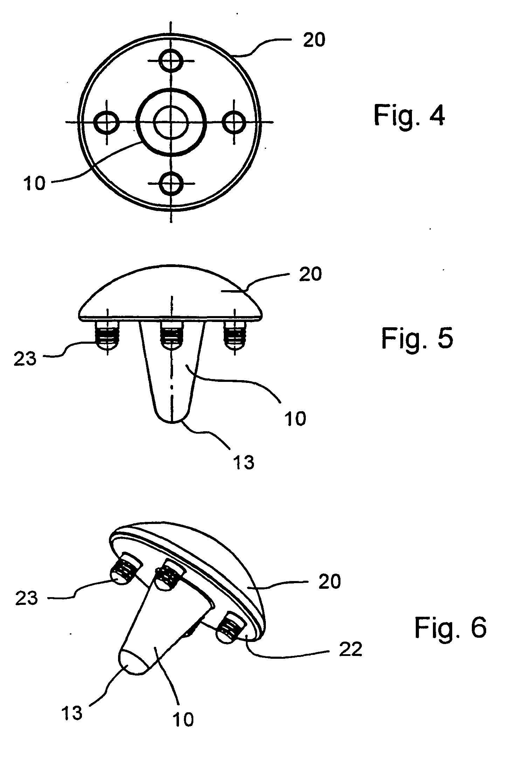

Die Figuren 4 bis 6 zeigen ebenfalls ein Ausführungsbeispiel einer einteiligen Gelenk-Endoprothese. Die Oberfläche des Schaftes 10 ist hierbei konisch und glatt. Die Spitze 13 ist ebenfalls abgerundet. Die Kopfkalotte 20 hat wiederum die Form eines Kugelabschnitts. Von der Unterseite 22 stehen nach unten vier Zapfen 23 ab, die die Endoprothese gegen Schubkräfte und Rotation sichern. Die Zapfen 23 haben eine Länge von etwa 5 bis 20 mm, einen Durchmesser von etwa 4 bis 10 mm und sind auf ihrer Oberfläche mit Rillen oder Gewinde versehen und/oder beschichtet, wodurch eine bessere Verankerung der Zapfen in entsprechenden Bohrungen der Spongiosa erzielt wird.

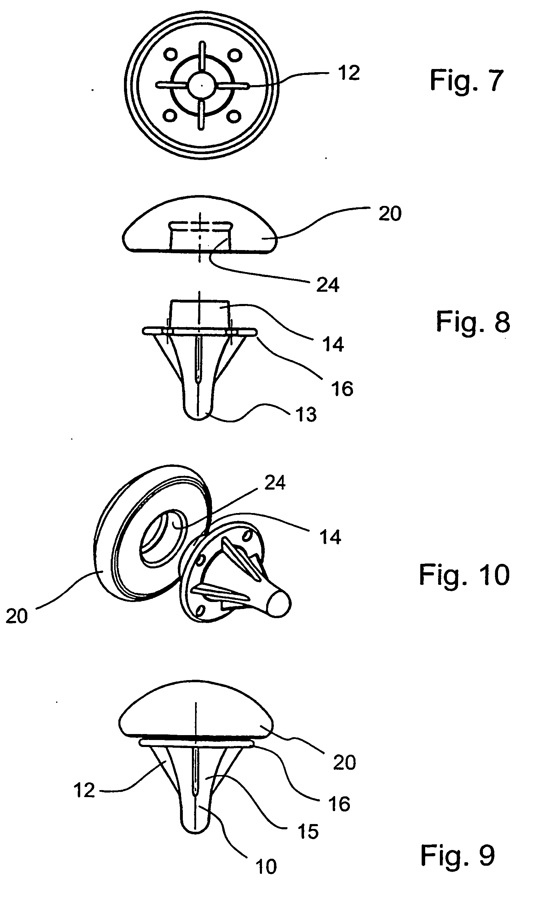

Die Figuren 7 bis 10 zeigen ein Ausführungsbeispiel einer zweiteiligen Gelenk-Endoprothese, wobei der Schaft 10 und die Kopfkalotte 20 als getrennte Elemente ausgebildet sind, die mittels eines Konus 14 am oberen Ende des Schafts 10 und einer entsprechenden konischen Aussparung 24 in der Unterseite der Kopfkalotte 20 zusammengefügt werden können. Der Schaft 10 setzt sich aus einem axialen Zapfen 15 zusammen, der sich mit einer konkaven Oberfläche zur stumpfen Spitze 13 hin verjüngt. Zwischen dem Konus 14 und dem Zapfen 15 ist eine quer zur Längsachse liegende Stützscheibe 16 ausgebildet. Zwischen der Stützscheibe 16 und dem Zapfen 15 sind gleichmäßig auf den Umfang verteilt vier Rippen 12 vorgesehen, deren Außenkanten sich unter einem Winkel von etwa 20 bis 70° zusammenlaufen, vorzugsweise etwa 60°.

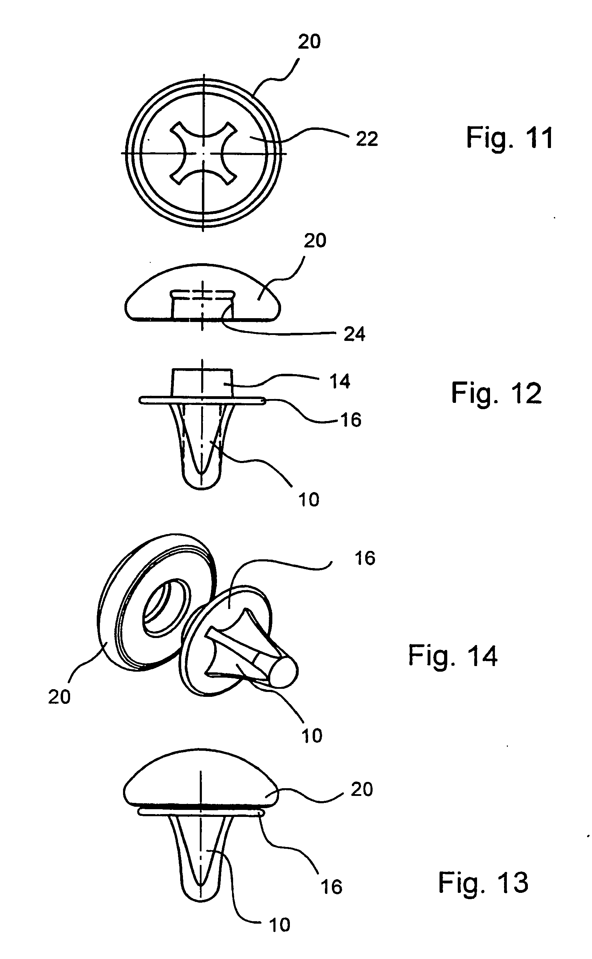

Die Fig. 11 bis 13 zeigen ein weiteres Ausführungsbeispiel einer zweiteiligen Gelenk-Endoprothese. Der Schaft 10 ist hierbei ähnlich wie der des Ausführungsbeispiels der Fig. 1 bis 3 kanneliert. Die Verbindung zwischen dem Schaft 10 und der Kopfkalotte wird wie bei dem Ausführungsbeispiel der Fig. 7 bis 10 durch einen Konus 14 am oberen Ende des Schaftes 10 und eine entsprechend konische Aussparung und eine Kopfkalotte 20 hergestellt. Zwischen dem Schaft 10 und dem Konus 14 ist auch hier eine Stützscheibe 16 vorgesehen.

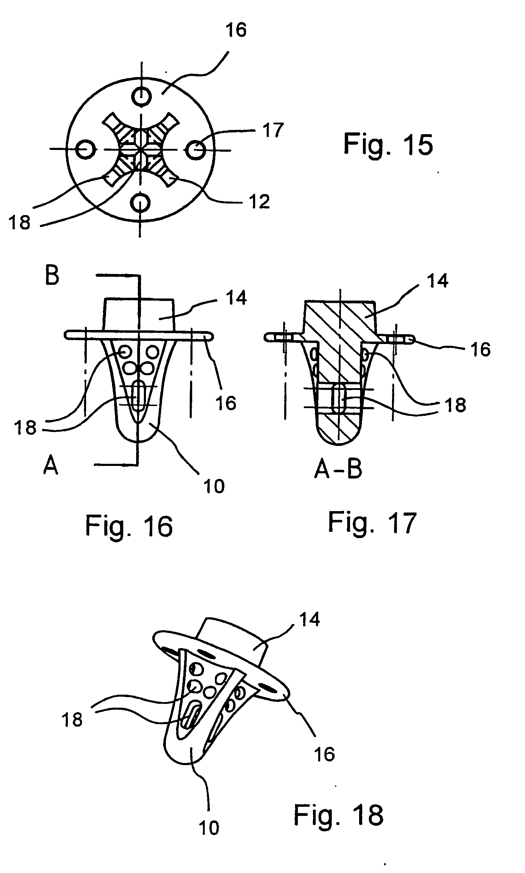

Die Fig. 15 bis 18 zeigen den Schaft 10 einer zweiteiligen Gelenk-Endoprothese ähnlich der der Fig. 11 bis 14, wobei der Schaft 10 mit Durchbrechungen 18 von kreisförmigem oder länglichem Querschnitt versehen ist. Die Durchbrechungen 18 ermöglichen ein Einwachsen der Knochensubstanz.

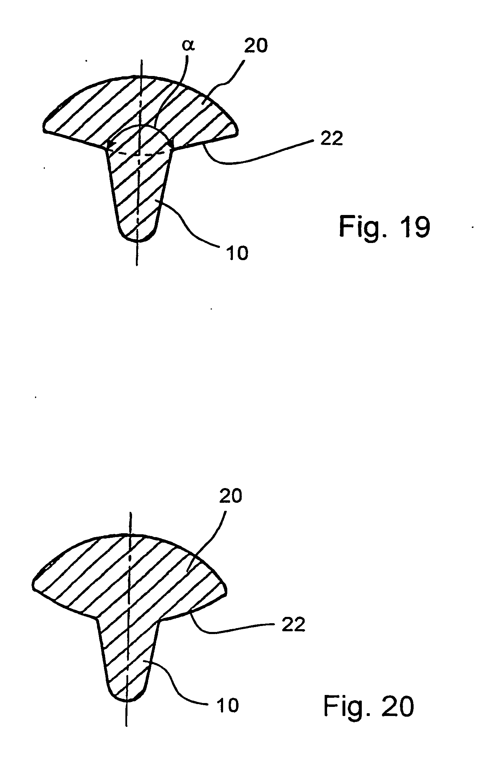

Die Fig. 19 und 20 zeigen im Schnitt Gelenk-Endoprothesen mit einer Kopfkalotte 20 und einem kurzen Schaft 10, wobei die Unterseite 22 der Kopfkalotte 20 konvex ist und nach unten flach konisch (Fig. 19) bzw. nach unten oder oben gewölbt ist (Fig. 20). Die Kopfkalotte 20 hat dadurch insgesamt eine Linsenform. Durch die konvexe Form der Unterseite 22 ergibt sich eine besonders homogene Einleitung der vom Gelenk ausgehenden Kräfte auf die Kortikalis und die Spongiosa.

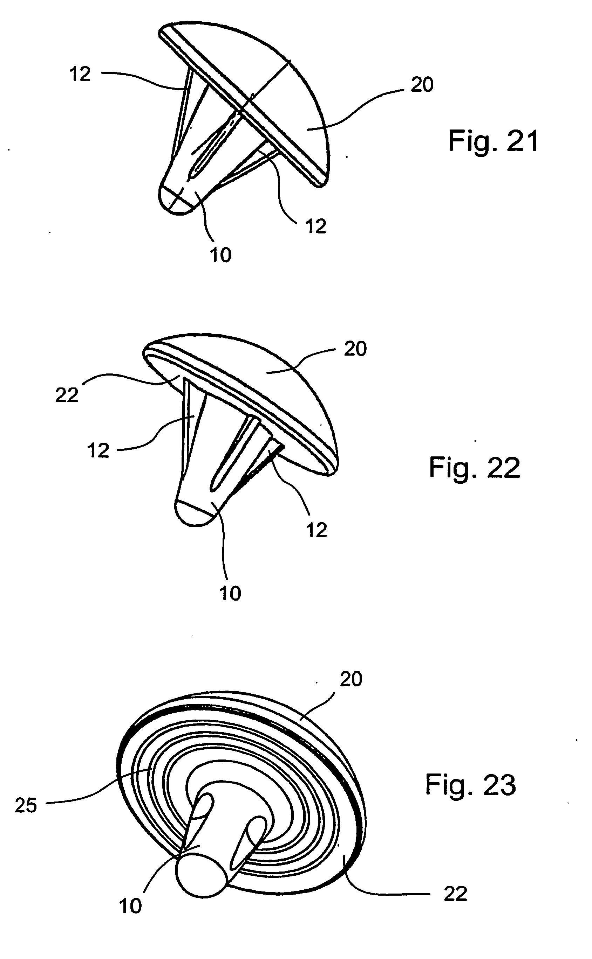

Die Fig. 21 und 22 zeigen einteilige Gelenk-Endoprothesen ähnlich der von Fig. 1 bis 3, wobei der Schaft 10 jedoch unter einem Winkel von etwa 10° zur Achse der Kopfkalotte 20 geneigt ist. Der Schaft ist so wie bei der Ausführungsform der Fig. 7 bis 9 mit radialen Rippen 12 versehen. Das Ansetzen des Schaftes 10 unter einem Winkel zur Kopfkalotte 20 kann bisweilen zweckmäßig sein, um zu verhindern, dass der Schaft die der Resektionsebene gegenüberliegende Kortikalis berührt.

Fig. 23 zeigt schräg von unten eine Endoprothese, bei der die Unterseite der Kopfkalotten 20 eine spiralförmig auslaufende Rille 25 aufweist. Die Rille 25 ermöglicht ein besseres Einwachsen des Knochens.

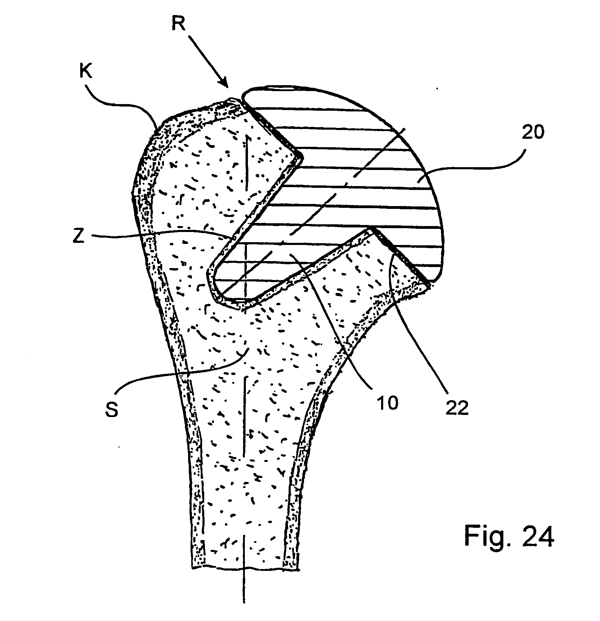

Fig. 24 zeigt im Schnitt die implantierte Gelenk-Endoprothese. Die ebene Unterseite 22 der Kopfkalotte 20 sitzt plan auf der Resektionsebene R auf und der Schaft erstreckt sich in die Spongiosa S, wobei seine Spitze einen ausreichenden Abstand von der Kortikalis K hält. Zwischen der Unterseite 22 der Kopfkalotte 20 und der Resektionsebene und zwischen dem Schaft 10 und der Spongiosa S ist ein schmaler Bereich 2 in Fig. 24 erkennbar, bei dem es sich um einen Zementköcher oder um eine Beschichtung der Endoprothese handeln kann. Ein solcher Zementköcher oder eine solche Beschichtung können vorhanden sein. Sie sind jedoch nicht in jedem Fall erforderlich.

### Bezugszeichenliste

- 10: Schaft
- 11: Rillen
- 12: Rippen
- 13: Spitze
- 14: Konus
- 15: Zapfen
- 16: Stützscheibe
- 18: Durchbrechung
- 20: Kopfkalotte
- 21: Oberseite
- 22: Unterseite
- 23: Zapfen
- 24: Aussparung
- 25: Rille

## Patentansprüche

1. Hüft- oder Schultergelenk-Endoprothese mit einem Schaft (10) und einer Kopfkalotte (20), die einteilig mit dem Schaft (10) ist oder am Schaft (10) befestigbar ist und eine gewölbte Oberseite (21) und eine geschlossene Unterseite (22) aufweist, wobei der Schaft (10) und die Kopfkalotte (20) jeweils eine Längsachse haben und die Achse der Kopfkalotte (20) durch den Kalottenmittelpunkt und senkrecht zur Unterseite (22) verläuft,
und wobei die Längsachse des Schaftes (10) einen Winkel von maximal 30° mit der Achse der Kopfkalotte (20) einschließt,
wobei sich der Schaft (10) mit einem Gesamtöffnungswinkel im Bereich von 5 bis 30° verjüngt,
wobei die Kopfkalotte (20) die Form eines Kugelabschnitts von weniger als einer Halbkugel hat und
wobei die Länge des Schaftes (10) kleiner als der Durchmesser der Kopfkalotte (20) ist,
**dadurch gekennzeichnet,**
**dass** zur Rotationssicherung eine oder mehrere Rippen (12) an dem konischen Schaft (10) ausgebildet sind, wobei sich die Rippen (12) radial um die Längsrichtung des konischen Schaftes (10) erstrecken und ein sicherer Sitz des konischen Schaftes durch seine Klemmung in der Spongiosa erreicht wird.

2. Endoprothese nach Anspruch 1,
**dadurch gekennzeichnet,**
**dass** die Längsachse des Schaftes (10) mit der Achse der Kopfkalotte (20) zusammenfällt.

3. Endoprothese nach Anspruch 1 oder 2,
**dadurch gekennzeichnet,**
**dass** sich der Schaft (10) mit einem Gesamtöffnungswinkel von etwa 20° verjüngt.

4. Endoprothese nach einem der Ansprüche 1 bis 3,
**dadurch gekennzeichnet,**
**dass** die Endoprothese zweiteilig ist, wobei der Schaft (10) und die Kopfkalotte (20) getrennte Teile sind, die miteinander verbindbar sind.

5. Endoprothese nach Anspruch 4,
**dadurch gekennzeichnet,**
**dass** der Schaft (10) und die Kopfkalotte (20) durch Formschluss oder Formkraftschluss miteinander verbindbar sind.

6. Endoprothese nach Anspruch 4,
**dadurch gekennzeichnet,**
**dass** die Verbindung von Schaft (10) und Kopfkalotte (20) über einen konischen Ansatz (14) am Schaft (10) und eine entsprechende konische Ausnehmung (24) in der Kopfkalotte (20) oder umgekehrt erfolgt.

7. Endoprothese nach einem der Ansprüche 4 bis 6,
**dadurch gekennzeichnet,**
**dass** der Schaft (10) an seinem der Kopfkalotte (20) zugewandten Ende zu einer Stützscheibe (16) verbreitet ist, an der die Kopfkalotte (20) anliegt.

8. Endoprothese nach einem der vorausgehenden Ansprüche,
**dadurch gekennzeichnet,**
**dass** sich der Schaft (10) ausgehend von der Unterseite (22) der Kopfkalotte (20) bzw. Stützscheibe (16) konisch oder über einen Radius in Richtung Schaftspitze (13) verjüngt.

9. Endoprothese nach einem der vorausgehenden Ansprüche,
**dadurch gekennzeichnet,**
**dass** der Schaft (10) eine oder mehrere Ausnehmungen oder Durchbrechungen (18), z.B. axial radial oder schräg verlaufende Fräsungen, Bohrungen oder Langlöcher aufweist.

10. Endoprothese nach einem der vorausgehenden Ansprüche,
**dadurch gekennzeichnet,**
**dass** die Unterseite (22) der Kopfkalotte (20) bzw. der Stützscheibe (16) eben ist.

11. Endoprothese nach einem der Ansprüche 1 bis 11,
**dadurch gekennzeichnet,**
**dass** die Unterseite (22) der Kopfkalotte (20) bzw. der Stützscheibe (16) flach-konisch, gewölbt-konvex oder gewölbt-konkav ist.

## Claims

1. A hip or shoulder joint endoprosthesis having a stem (10) and a head cap (20), which is in one piece with the stem (10) or is attachable to the stem (10) and comprises a curved upper side (21) and a closed underside (22), the stem (10) and the head cap (20) each having a longitudinal axis and the axis of the head cap (20) extending through the centre of the cap and perpendicularly to the underside (22),
and the longitudinal axis of the stem (10) forming an angle of at most 30° with the axis of the head cap (20),
the stem (10) tapering with a total angle of taper ranging from 5 to 30°,
the head cap (20) having the shape of a portion of a sphere smaller than a hemisphere and
the length of the stem (10) being shorter than the diameter of the head cap (20),
**characterised in that**
one or more ribs (12) are formed on the conical stem (10) to secure against rotation, the ribs (12) extending radially about the longitudinal direction of the conical stem (10) and secure seating of the conical stem being achieved by wedging thereof in the cancellous bone.

2. An endoprosthesis according to claim 1,
**characterised in that**
the longitudinal axis of the stem (10) coincides with the axis of the head cap (20).

3. An endoprosthesis according to claim 1 or claim 2,
**characterised in that**
the stem (10) tapers with a total angle of taper of approximately 20°.

4. An endoprosthesis according to one of claims 1 to 3,
**characterised in that**
the endoprosthesis is in two parts, the stem (10) and the head cap (20) being separate parts which may be connected together.

5. An endoprosthesis according to claim 4,
**characterised in that**
the stem (10) and the head cap (20) may be connected together positively or positively and non-positively.

6. An endoprosthesis according to claim 4,
**characterised in that**
the connection of stem (10) and head cap (20) takes place via a conical extension piece (14) on the stem (10) and a corresponding conical recess (24) in the head cap (20), or vice versa.

7. An endoprosthesis according to one of claims 4 to 6,
**characterised in that**
the stem (10) is widened at its end facing the head cap (20) to form a supporting disc (16), against which the head cap (20) rests.

8. An endoprosthesis according to one of the preceding claims,
**characterised in that**,
starting from the underside (22) of the head cap (20) or supporting disc (16), the stem (10) tapers conically or over a radius in the direction of the stem tip (13).

9. An endoprosthesis according to one of the preceding claims,
**characterised in that**
the stem (10) comprises one or more recesses or openings (18), e.g. axially, radially or obliquely extending milled holes, drilled holes or slots.

10. An endoprosthesis according to one of the preceding claims,
**characterised in that**
the underside (22) of the head cap (20) or of the supporting disc (16) is flat.

11. An endoprosthesis according to one of claims 1 to 11,
**characterised in that**
the underside (22) of the head cap (20) or of the supporting disc (16) is flat conical, curved convex or curved concave.

## Revendications

1. Endoprothèse d'articulation de la hanche ou de l'épaule, munie d'une tige (10) et d'une calotte de tête (20) qui est d'un tenant avec la tige (10) ou qui peut être fixée sur la tige (10) et qui présente une face supérieure bombée (21) et une face inférieure fermée (22), la tige (10) et la calotte de tête (20) possédant respectivement un axe longitudinal et l'axe de la calotte de tête (20) passant par le centre de la calotte et de manière perpendiculaire par rapport à la face inférieure (22),
et l'axe longitudinal de la tige (10) incluant un angle de 30° maximal avec l'axe de la calotte de tête (20),
la tige (10) s'effilant avec un angle d'ouverture total de l'ordre de 5 à 30°,
la calotte de tête (20) ayant la forme d'un segment sphérique inférieur à une hémisphère et
la longueur de la tige (10) étant inférieure au diamètre de la calotte de tête (20),
**caractérisée en ce**
**que** pour assurer la rotation une ou plusieurs nervures (12) sont formées sur la tige conique (10), les nervures (12) s'étendant dans le sens radial autour de la direction longitudinale de la tige conique (10) et un logement sûr de la tige conique étant obtenu par son serrage dans le tissu osseux spongieux.

2. Endoprothèse selon la revendication 1,
**caractérisée en ce**
**que** l'axe longitudinal de la tige (10) coïncide avec l'axe de la calotte de tête (20).

3. Endoprothèse selon la revendication 1 ou 2,
**caractérisée en ce**
**que** la tige (10) s'effile avec un angle d'ouverture total d'environ 20°.

4. Endoprothèse selon l'une quelconque des revendications 1 à 3,
**caractérisée en ce**
**que** l'endoprothèse est en deux parties, la tige (10) et la calotte de tête (20) étant des parties séparées, qui peuvent être reliées l'une à l'autre.

5. Endoprothèse selon la revendication 4,
**caractérisée en ce**
**que** la tige (10) et la calotte de tête (20) peuvent être reliées l'une à l'autre par correspondance de forme ou par adhérence de forme.

6. Endoprothèse selon la revendication 4,
**caractérisée en ce**
**que** la liaison de la tige (10) et de la calotte de tête (20) se fait par l'intermédiaire d'un téton conique (14) sur la tige (10) et par un évidement conique correspondant (24) dans la calotte de tête (20), ou inversement.

7. Endoprothèse selon l'une quelconque des revendications 4 à 6,
**caractérisée en ce**
**que** la tige (10) est élargie à son extrémité tournée vers la calotte de tête (20) pour former un disque d'appui (16), sur lequel s'appuie la calotte de tête (20).

8. Endoprothèse selon l'une quelconque des revendications précédentes,
**caractérisée en ce**
**que** partant de la face inférieure (22) de la calotte de tête (20) et/ou du disque d'appui (16), la tige (10) s'effile de manière conique ou au moyen d'un rayon en direction de la pointe de la tige (13).

9. Endoprothèse selon l'une quelconque des revendications précédentes,
**caractérisée en ce**
**que** la tige (10) présente un ou plusieurs évidements ou perçages (18), par exemple des fraisages, des alésages ou des trous oblongs s'étendant axialement, radialement ou obliquement.

10. Endoprothèse selon l'une quelconque des revendications précédentes,
**caractérisée en ce**
**que** la face inférieure (22) de la calotte de tête (20) et/ou du disque d'appui (16) est plane.

11. Endoprothèse selon l'une quelconque des revendications 1 à 11,
**caractérisée en ce**
**que** la face inférieure (22) de la calotte de tête (20) et/ou du disque d'appui (16) est conique plate, bombée convexe ou bombée concave.
